# DEMANDE DE BREVET EUROPEEN

(11) **EP 0 852 262 A1**
(43) Date de publication de la demande: **08.07.1998**
(21) Numéro de dépôt: 97420225.1
(22) Date de dépôt: 04.12.1997
(51) Int. Cl.: C12P 13/02, C07C 233/09

(54) **Procédé de préparation d'amides de l'acide acrylique ou méthacrylique**

(30) Priorité: 13.12.1996 FR 9615612
(71) Demandeur: COATEX S.A., F-69730 Genay (FR)
(72) Inventeur: Egraz, Jean-Bernard, 69130 Ecully (FR); Suau, Jean Marc, 69480 Lucenay (FR); Brigodiot-Ignazi, Maryvonne, 92290 Chatenay-Malabry (FR); Peixoto, Isabelle, 75011 Paris (FR); Lalot, Thierry, 91170 Viry-Chatillon (FR)

(57) **Abrégé**

Procédé de préparation d'amides de l'acide acrylique ou méthacrylique fonctionnalisés par une amine tertiaire cationisable, par aminolyse, à l'aide d'enzymes, d'esters de l'acide acrylique ou méthacrylique.

## Description

La présente invention concerne un procédé de préparation, par aminolyse d'esters de l'acide acrylique ou méthacrylique à l'aide d'enzymes, d'amides fonctionnalisés par une amine tertiaire cationisable de l'acide acrylique ou méthacrylique.

L'invention concerne également les amides de l'acide acrylique ou méthacrylique obtenus par le dit procédé.

Depuis longtemps déjà l'homme de l'art connaît divers procédés permettant d'obtenir des amides acryliques à partir des esters en question par réaction avec les amines correspondantes pour éviter ou minimiser la réaction secondaire dite de Michaël résultant de l'addition de l'amine sur la double liaison acrylique ou méthacrylique.

Ainsi le brevet FR 2 259 088 révèle un procédé en plusieurs étapes passant par la formation d'un produit intermédaire tel que le β-amino-propionamide qui est ensuite traité thermiquement à de hautes températures.
Ce procédé connu présente l'inconvénient de générer des réactions de décompositions indésirables et d'avoir un rendement global faible.

Une autre solution connue (FR 2 423 482) consiste à utiliser un catalyseur à base d'oxydes d'étain dialkylés accélérateur de la réaction d'aminolyse. Mais un tel procédé fait appel à des composés ne répondant pas aux préoccupations actuelles de la réglementation en matière d'environnement et d'écologie.

Pour pallier aux différents problèmes exposés ci-dessus, une autre méthode connue (FR 2 590 567) propose un procédé faisant réagir un anhydride acrylique ou méthacrylique sur une diamine. Mais une telle méthode a le désavantage de partir d'un anhydride onéreux, difficile d'approvisionnement et générant comme sous produit de l'acide acrylique ou méthacrylique.

Confrontée à ces divers inconvénients, la Demanderesse a mis au point un procédé en une seule étape permettant l'obtention d'amides de l'acide acrylique ou méthacrylique fonctionnalisés par une amine tertiaire cationisable avec un rendement global élevé et de faibles taux de réactions secondaires telles que la polymérisation ou la réaction de Michaël tout en mettant en oeuvre des conditions opératoires et des composés respectant les normes et/ou les règlementations actuelles de l'environnement.

Ainsi un des buts de l'invention est la mise au point dudit procédé permettant la synthèse du monomère et limitant la formation des produits résultant de la réaction de Michaël par aminolyse des esters acryliques ou méthacryliques en présence d'au moins un inhibiteur de polymérisation et à l'aide d'enzymes.

Un autre but de l'invention concerne les amides de l'acide acrylique ou méthacrylique fonctionnalisés par une amine tertiaire cationisable et obtenus par le dit procédé et plus précisément les acrylamides ou méthacrylamide de N-dialkylamino alkyle ou oxyalkyle de formule (I) : 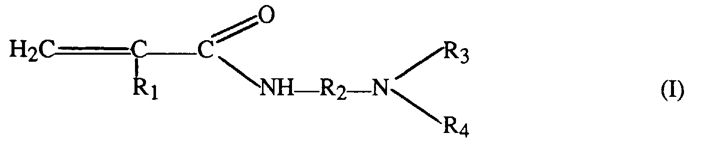 dans laquelle
- R₁ est un atome d'hydrogène ou un radical méthyle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène issus par exemple de l'oxyde d'éthylène ou de propylène ou de leurs mélanges.
- R₃ et R₄ sont deux radicaux identiques ou différents choisis parmi les groupes alkyle linéaires ou ramifiés pouvant contenir jusqu'à 18 atomes de carbone.

Ces buts sont atteints en faisant réagir des esters acryliques ou méthacryliques et plus particulièrement l'acrylate ou méthacrylate de méthyle ou d'éthyle avec une diamine de formule générale : 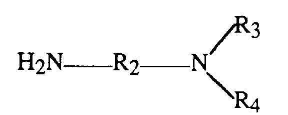 en présence d'au moins un inhibiteur de polymérisation et à l'aide d'enzymes.

Dans le procédé selon l'invention, l'inhibiteur de polymérisation peut être choisi parmi tous les inhibiteurs de polymérisation bien connus de l'homme du métier et notamment parmi l'alloocimène, l'hydroquinone, l'éther méthylique de l'hydroquinone, la phénothiazine, le 2,6-terbutyl-p-crésol.

Ainsi le procédé, selon l'invention, d'obtention d'amides de l'acide acrylique ou méthacrylique fonctionnalisés par une amine tertiaire cationisable de formule générale (I) se caractérise en ce que l'on fait réagir en présence d'au moins un inhibiteur de polymérisation un ester acrylique ou méthacrylique d'alkyle de formule: 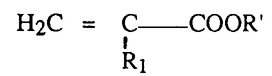 dans laquelle
- R₁ est un atome d'hydrogène ou un radical méthyle
- R' est un radical alkyle linéaire ou ramifié et plus particulièrement le radical méthyle ou éthyle
avec une diamine de formule générale : 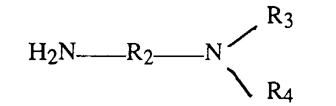 dans laquelle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène choisis parmi les motifs d'oxyde d'éthylène ou de propylène ou de leurs mélanges.
- R₃ et R₄ sont deux radicaux identiques ou différents choisis parmi les groupes alkyle linéaires ou ramifiés pouvant contenir jusqu'à 18 atomes de carbone
à l'aide d'une ou plusieurs enzymes dont le ou les sites actifs reconnaissent la fonction ester dudit ester acrylique ou méthacrylique.

Parmi ces enzymes on peut notamment citer les lipases de diverses origines, comme par exemple d'origine fongique ou bactérienne ou bien encore animale.

Parmi les lipases d'origine bactérienne ou fongique, on peut citer les lipases de Chromobacterium viscosum, d'Aspergillus niger, de Mucor miehei, de Rhizopus arrhizus, de Candida cylindracea, de Candida antarctica, de Rhizopus delemar, de Mucor javanicus, de Pseudomonas fluorescens. Comme lipase d'origine animale, on peut nommer la lipase de pancréas de porc.

De manière plus particulière la ou les enzymes utilisées dans le procédé selon l'invention sont choisies parmi une lipase de Candida antarctica immobilisée sur une résine macroporeuse de type acrylique connue sous la dénomination Novozyme® ou bien encore parmi une lipase de Mucor miehei connue sous la dénomination Lipozyme® ou bien enfin leurs mélanges.

Les esters acryliques et méthacryliques sont choisis généralement parmi les acrylates ou méthacrylates d'alkyle et plus particulièrement parmi l'acrylate de méthyle, l'acrylate d'éthyle, le méthacrylate de méthyle, le méthacrylate d'éthyle.

Les amines utilisées sont généralement des amines primaires fonctionnalisées par des amines tertiaires donnant naissance à des diamines parmi lesquelles on peut citer les dialkylamino alkyle ou oxyalkyle de formule : 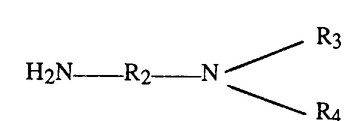 dans laquelle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène issus par exemple de l'oxyde d'éthylène ou de propylène ou de leurs mélanges.
- R₃ et R₄ sont deux radicaux identiques ou différents choisis parmi les groupes alkyle linéaires ou ramifiés pouvant contenir jusqu'à 18 atomes de carbone.

De plus la réaction selon l'invention est réalisée à pression atmosphérique et à une température comprise entre 20°C et 100°C et préférentiellement entre 40°C et 90°C.

Le produit brut de réaction obtenu après refroidissement contient l'acrylamide ou le méthacrylamide de N-dialkylamino alkyle ou oxyalkyle de formule (I) correspondant à l'ester de départ, l'excès de diamine et/ou d'ester n'ayant pas réagi, les produits de la réaction de Michaël ainsi que la ou les enzymes mises en oeuvre au cours de la réaction, ces enzymes étant récupérées par filtration.

La composition du produit brut obtenu est déterminée qualitativement et quantitativement par l'analyse RMN ¹ H dudit produit brut en solution dans le chloroforme deutérié.

Ainsi un mode de réalisation préféré de l'invention met en oeuvre une réaction d'aminolyse du méthacrylate de méthyle par la 3-diméthylaminopropylamine en présence de lipases du type Novozyme® ou Lipozyme® pour obtenir le 3-diméthylaminopropylméthacrylamide.

La portée et l'intérêt de l'invention seront mieux perçus grâce aux exemples suivants qui ne sauraient présenter un quelconque caractère limitatif.

### Exemple 1 :

Dans un réacteur d'un litre muni d'un système d'agitation mécanique, d'un réfrigérant et d'un système de chauffage à bain d'huile, on charge sous agitation et à température ambiante 901,6 g de méthacrylate de méthyle, 0,9 g d'alloocimène et 80,1 g de lipase de Candida antarctica commercialisée par la société Novobioindustrie sous le nom de Novozyme® d'activité de 7400 PLU/g (PLU = Propyl Laurate Units).

Après une montée de la température à 60°C, on ajoute 119,4 g de 3-diméthylaminopropylamine de manière continue pendant 9 heures à l'aide d'une canne plongeante en inox et sous un bullage d'air ayant un débit de 180 litres/heure ainsi que 988,7 g de méthacrylate de méthyle correspondant à la masse de méthacrylate de méthyle entraîné par le dit bullage.

Il est à noter que le bullage d'air déplace l'équilibre par entraînement du méthanol formé par la réaction d'aminolyse et empêche la polymérisation des monomères.

A la fin de la période d'injection, le chauffage est coupé et l'agitation maintenue pendant une heure.

Le produit alors obtenu est refroidi à température ambiante puis filtré. Le filtrat est une solution constituée de méthacrylate de méthyle, d'amine n'ayant pas réagi, de méthacrylamide formé ainsi que des produits de la réaction de Michaël.
Les rendements sont déterminés par rapport à l'amine engagée à l'aide de l'analyse des spectres RMN ¹ H réalisés sur les solutions du filtrat dans le chloroforme deutérié.

Ainsi pour cet essai, le filtrat contient 97 % de 3-diméthylaminopropylméthacrylamide et 3 % de produits de Michaël aboutissant ainsi à une forte conversion en amide (97 %) et à une faible formation de produits de Michaël (3 %).

### Exemple 2 :

Dans un réacteur identique à celui décrit dans l'exemple précédent, on introduit à température ambiante 903,4 g de méthacrylate de méthyle, 1,1 g d'alloocimène, 80 g de la même lipase que celle utilisée dans l'exemple précédent.

Après une montée de la température à 60°C, on ajoute 127,3 g de la même diamine que celle décrite dans l'exemple 1 de manière continue pendant 15 heures à l'aide d'une canne plongeante en inox, sous un bullage d'air mis en oeuvre à l'aide d'un diffuseur à gaz, à un débit d'environ 50 litres/heure. 82 g de méthacrylate de méthyle correspondant à la masse de méthacrylate de méthyle entraîné par le dit bullage sont ajoutés lors de la réaction pour maintenir constant le volume réactionnel.

A la fin de la période d'injection, le chauffage est coupé et l'agitation maintenue pendant une heure.

Le produit obtenu est alors refroidi à température ambiante puis filtré.

Le filtrat est une solution à 90 % de 3-diméthylaminopropylméthacrylamide déterminé à l'aide de l'analyse des spectres RMN ¹ H réalisés sur le filtrat en solution dans le chloroforme deutérié montrant ainsi qu'il est possible de diminuer les débits d'air et d'amine tout en conservant un fort taux de conversion en amide (90 %) et une faible teneur en produits de Michaël (10 %).

### Exemple 3 :

Dans cet exemple on fait réagir les mêmes composés que ceux utilisés dans l'exemple 1 en présence de la moitié de la quantité de Novozyme® par rapport à l'amine.

Ainsi, dans un appareillage identique à celui décrit dans l'exemple 1, on introduit, sous agitation et à température ambiante, 450 g de méthacrylate de méthyle, 0,45 g d'alloocimène et 40,1 g de lipase de Candida antarctica commercialisée par la société Novobioindustrie sous le nom de Novozyme® d'activité de 7400 PLU/g (PLU = Propyl Laurate Units).

Après une montée de la température à 60°C, on ajoute 123,6 g de 3-diméthylaminopropylamine de manière continue pendant 18 heures à l'aide d'une canne plongeante en inox et sous un bullage d'air ayant un débit de 180 litres/heure ainsi que 555,5 g de méthacrylate de méthyle correspondant à la masse de méthacrylate de méthyle entraîné par le dit bullage.
Il est à noter que le bullage d'air déplace l'équilibre par entraînement du méthanol formé lors de la réaction d'aminolyse et empêche la polymérisation des monomères.

A la fin de la période d'injection, le chauffage est coupé et l'agitation maintenue pendant une heure.

On obtient alors un filtrat contenant 92 % de méthacrylamide de diméthylaminopropyle, 6 % de produits de Michaël et 2 % d'amine résiduelle, composition déterminée à l'aide de l'analyse des spectres RMN ¹ H réalisés sur le filtrat en solution dans le chloroforme deutérié donnant ainsi une forte conversion en amide (92 %) et une faible formation de produits de Michaël (6 %).

### Exemple 4 :

Dans cet exemple, la réaction d'aminolyse de l'ester s'effectue à 40°C.

Ainsi dans un appareillage identique à celui décrit dans l'exemple 1, on introduit, sous agitation et à température ambiante, 900,7 g de méthacrylate de méthyle, 0,9 g d'alloocimène et 80,1 g de lipase de Candida antarctica commercialisée par la société Novobioindustrie sous le nom de Novozyme® d'activité de 7400 PLU/g (PLU = Propyl Laurate Units).

Après une montée de la température à 40°C, on ajoute 123,6 g de 3-diméthylaminopropylamine de manière continue pendant 9 heures à l'aide d'une canne plongeante en inox et sous un bullage d'air ayant un débit de 180 litres/heure ainsi que 555,5 g de méthacrylate de méthyle correspondant à la masse de méthacrylate de méthyle entraîné par le dit bullage.

Il est à noter que le bullage d'air déplace l'équilibre par entraînement du méthanol formé lors de la réaction d'aminolyse et empêche la polymérisation des monomères.

A la fin de la période d'injection, le chauffage est coupé et l'agitation maintenue pendant une heure.

On obtient alors un filtrat dont la composition déterminée à l'aide de l'analyse des spectres RMN ¹ H réalisés sur le filtrat en solution dans le chloroforme deutérié est : 79 % de 3-diméthylaminopropylméthacrylamide, 17 % de produits de Michaël et 4 % d'amine résiduelle, donnant ainsi une forte conversion en amide (79 %) avec une formation de produits de Michaël de l'ordre de 17 %.

### Exemple 5 :

Dans cet exemple, la réaction d'aminolyse de l'ester s'effectue à 90°C.

Pour des quantités de réactifs identiques à celles de l'exemple précédent ainsi qu'avec un mode opératoire semblable à celui décrit dans l'exemple précédent à l'exception de la température de réaction qui est de 90°C on obtient un filtrat dont la composition, déterminée à l'aide de l'analyse des spectres RMN ¹ H réalisés sur le filtrat en solution dans le chloroforme deutérié, est 96 % de 3-diméthylaminopropylméthacrylamide et 4 % de produits de Michaël aboutissant ainsi à une forte conversion en amide (96 %) et une faible formation de produits de Michaël (4 %).

### Exemple 6 :

Dans un réacteur de 0,2 litre à double paroi muni d'un système d'agitation magnétique et d'un système de chauffage à circulation d'huile, on charge sous agitation et à température ambiante 47,2 g de méthacrylate de méthyle, 0,059 g d'hydroquinone monométhyléther et 9 g de lipase de Mucor miehei commercialisée par la société Novobioindustrie sous le nom de Lipozyme® d'activité de 7,7 BAUN/g (BAUN = Batch Acidolysis Unit Novo) ou d'activité de 45 BIU/g (BIU = Batch Interesterification Units).

Après une montée en température à 40°C, on ajoute 7,3 g de 3-diméthylaminopropylamine de manière continue pendant 6 heures à l'aide d'une ampoule à brome et sous bullage d'air ayant un débit de 180 litres/heure ainsi que 8,5 g de méthacrylate de méthyle correspondant à la masse de méthacrylate de méthyle entraîné par ledit bullage.

A la fin de la période d'injection, le chauffage est coupé et l'agitation maintenue pendant trente minutes.

Le produit alors obtenu est refroidi à température ambiante puis filtré. Le filtrat est une solution constituée de méthacrylate de méthyle, d'amine n'ayant pas réagi, de méthacrylamide formé ainsi que des produits de la réaction de Michaël.
Les calculs de rendement sont déterminés à l'aide de l'analyse des spectres RMN ¹ H réalisés sur le filtrat en solution dans le chloroforme deutérié
Ainsi pour cet essai, le filtrat contient 83 % de 3-diméthylaminopropylméthacrylamide, 16 % de produits de Michaël et 1 % d'amine résiduelle aboutissant ainsi à une forte conversion en amide (83 %).

### Exemple 7 :

Dans cet exemple, on fait réagir avec le même mode opératoire et le même appareillage que ceux décrits dans l'exemple 1, 901 g d'acrylate de méthyle, 0,9 g d'alloocimène, 80 g de lipase commercialisée sous le nom de Novozyme® d'activité de 7400 PLU/g et 119 g de 3-diméthylaminopropylamine.

Le produit alors obtenu contient 97 % de 3-diméthylaminopropylacrylamide et 3 % de produits de Michaël déterminé à l'aide de l'analyse des spectres RMN ¹ H réalisés sur le filtrat en solution dans le chloroforme deutérié.

### Exemple 8 :

Dans cet exemple, on reproduit l'exemple 1.

On obtient comme pour l'exemple 1, un filtrat de composition identique à celle de l'exemple 1.

On récupère alors totalement l'enzyme mise en oeuvre dans cet essai, enzyme que l'on réutilise dans une opération en tout point identique à la précédente.

Le filtrat obtenu contient alors 96 % de 3-diméthylaminopropylméthacrylamide et 4 % de produits de Michaël déterminé à l'aide de l'analyse des spectres RMN ¹ H réalisés sur le filtrat en solution dans le chloroforme deutérié

Une nouvelle récupération totale de l'enzyme et une nouvelle mise en oeuvre dans les mêmes conditions que précédemment permet d'aboutir à un produit ayant une composition de 94 % de 3-diméthylaminopropylméthacrylamide et 6 % de produits de Michaël déterminé à l'aide de l'analyse des spectres RMN ¹ H réalisés sur le filtrat en solution dans le chloroforme deutérié

Ceci étant, il est démontré qu'il est donc possible d'utiliser au moins 3 fois le même échantillon d'enzyme avec un rendement toujours supérieur ou égal à 94 %.

## Revendications

1. Procédé de préparation, par aminolyse d'esters de l'acide acrylique ou de l'acide méthacrylique, d'amides fonctionnnalisés par une amine tertiaire cationisable caractérisé en ce que l'on fait réagir un ester acrylique ou méthacrylique avec une diamine en présence d'au moins un inhibiteur de polymérisation et à l'aide d'une ou plusieurs enzymes dont le ou les sites actifs reconnaissent la fonction ester dudit ester acrylique ou méthacrylique.

2. Procédé de préparation selon la revendication 1 d'amides fonctionnalisés par une amine tertiaire cationisable de formule générale (I) : 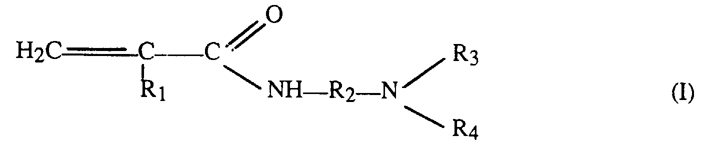 dans laquelle
- R₁ est un atome d'hydrogène ou un radical méthyle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène choisis parmi les motifs d'oxyde d'éthylène ou de propylène ou de leurs mélanges
- R₃ et R₄ sont deux radicaux identiques ou différents choisis parmi les groupes alkyle linéaires ou ramifiés pouvant contenir jusqu'à 18 atomes de carbone
caractérisé en ce que l'on fait réagir, en présence d'au moins un inhibiteur de polymérisation, un ester acrylique ou méthacrylique d'alkyle de formule : 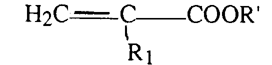 dans laquelle
- R₁ est un atome d'hydrogène ou un radical méthyle
- R' est un radical alkyle linéaire ou ramifié avec une diamine de formule générale : 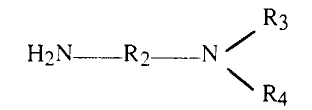 dans laquelle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène choisis parmi les motifs d'oxyde d'éthylène ou de propylène ou de leurs mélanges
- R₃ et R₄ sont deux radicaux identiques ou différents choisis parmi les groupes alkyle linéaires ou ramifiés pouvant contenir jusqu'à 18 atomes de carbone
à l'aide d'une ou plusieurs enzymes dont le ou les sites actifs reconnaissent la fonction ester dudit ester acrylique ou méthacrylique.

3. Procédé de préparation selon la revendication 2 d'amides fonctionnnalisés par une amine tertiaire cationisable de formule générale (I), caractérisé en ce que l'on fait réagir, en présence d'au moins un inhibiteur de polymérisation, un acrylate ou méthacrylate de méthyle ou d'éthyle, avec une diamine de formule générale : 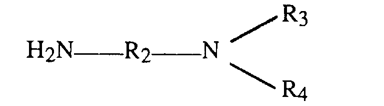 dans laquelle
- R₂ est un radical alkyle, linéaire ou ramifié pouvant contenir jusqu'à 20 atomes de carbone ou bien un radical oxyalkylé pouvant contenir jusqu'à 50 motifs d'oxyde d'alkylène choisis parmi les motifs d'oxyde d'éthylène ou de propylène ou de leurs mélanges
- R₃ et R₄ sont deux radicaux identiques ou différents choisis parmi les groupes alkyle linéaires ou ramifiés pouvant contenir jusqu'à 18 atomes de carbone
à l'aide d'une ou plusieurs enzymes dont le ou les sites actifs reconnaissent la fonction ester dudit acrylate ou méthacrylate.

4. Procédé de préparation selon la revendication 3 d'amides fonctionnalisés par une amine tertiaire cationisable de formule générale (I), caractérisé en ce que l'on fait réagir, en présence d'au moins un inhibiteur de polymérisation, le méthacrylate de méthyle avec la 3-diméthylaminopropylamine à l'aide d'une ou plusieurs enzymes dont le ou les sites actifs reconnaissent la fonction ester dudit méthacrylate.

5. Procédé de préparation selon l'une quelconque des revendications 1 à 4 d'amides fonctionnalisés par une amine tertiaire cationisable caractérisé en ce que la ou les enzymes sont constituées des lipases d'origine fongique, bactérienne ou animale.

6. Procédé de préparation selon la revendication 5 d'amides fonctionnalisés par une amine tertiaire cationisable caractérisé en ce que la ou les enzymes sont choisies parmi les lipases de Chromobacterium viscosum, d'Aspergillus niger, de Mucor miehei, de Rhizopus arrhizus, de Candida cylindracea, de Candida antarctica, de Rhizopus delemar, de Mucor javanicus, de Pseudomonas fluorescens, la lipase de pancréas de porc ou leurs mélanges.

7. Procédé de préparation selon la revendication 6 d'amides fonctionnalisés par une amine tertiaire cationisable caractérisé en ce que la ou les enzymes sont choisies parmi les lipases de Candida antarctica ou des lipases de Mucor miehei ou leurs mélanges.

8. Procédé de préparation selon l'une quelconque des revendications 1 à 7 d'amides fonctionnalisés par une amine tertiaire cationisable caractérisé en ce que la température de réaction est comprise entre 20°C et 100°C et préférentiellement entre 40°C et 90°C.

9. Amides d'acide acrylique ou méthacrylique obtenus par le procédé de préparation selon l'une quelconque des revendications 1 à 8.
